# EUROPÄISCHE PATENTSCHRIFT

(11) **EP 1 201 111 B1**
(45) Veröffentlichungstag und Bekanntmachung des Hinweises auf die Patenterteilung: **23.10.2002**
(21) Anmeldenummer: 00123038.2
(22) Anmeldetag: 24.10.2000
(51) Int. Cl.: A01C 3/02, C12M 1/113, C05F 17/02

(54) **Biogasanlagen-Zuführungseinrichtung**
Feeding device for biogas installation
Dispositif d'alimentation pour installation de biogaz

(43) Veröffentlichungstag der Anmeldung: 02.05.2002
(73) Patentinhaber: PlanET Energietechnik GmbH, 48691 Vreden (DE)
(72) Erfinder: Becker, Hendrik, Dipl.-Ing. (FH), 48691 Vreden (DE); Meyer zu Strohe, Jörg, Dipl.-Ing. (FH), 49565 Bramsche (DE)
(74) Vertreter: Rohmann, Michael, Dr.

(56) Entgegenhaltungen:
- CA-A- 2 080 547
- DE-A- 3 829 018
- FR-A- 2 612 834
- GB-A- 2 282 337
- US-A- 4 290 737
- US-A- 5 248 612

## Beschreibung

Die Erfindung betrifft eine Biogasanlagen-Zuführungseinrichtung für vergärbares organisches Material, wobei ein Vorlagebunker zur Aufnahme des organischen Materials vorgesehen ist, wobei unterhalb des Vorlagebunkers zumindest ein Zuführungskanal für eine Aufnahme des organischen Materials aus dem Vorlagebunker vorgesehen ist und wobei in dem Zuführungskanal ein Druckkolben angeordnet ist, unter dessen Einwirkung dass in dem Zuführungskanal aufgenommene organische Material der Biogasanlage zuführbar ist. - Die Erfindung befasst sich also mit einer Einrichtung zur Zuführung von vergärbarem organischem Material in eine Biogasanlage. Erfindungsgemäß handelt es sich insbesondere um festes vergärbares organisches Material oder um vergärbares organisches Material mit hohem Feststoffgehalt, der zweckmäßigerweise zwischen 15 und 75 Gew.-%, vorzugsweise zwischen 20 und 70 Gew.-% beträgt.

Bei Biogas handelt es sich um einen Energieträger, dessen Hauptkomponente das Methan ist. Es entsteht durch mikrobiellen Abbau vergärbarer organischer Substanzen. Unter dem Begriff Vergärung versteht man den Abbau von biogenem Material durch Mikroorganismen in Abwesenheit von Sauerstoff, das heißt unter anaeroben Bedingungen. Bakteriengruppen verwandeln das biogene Material in Biogas. Als vergärbares organisches Material eignen sich insbesondere feste biogene Abfälle bzw. biogene Abfälle mit hohem Feststoffanteil. Für die Vergärung eignen sich weiterhin aber auch Gülle und organisch belastete Abwässer. Während die Zuführung der letztgenannten flüssigen vergärbaren Substanzen verhältnismäßig einfach ist, ist die dosierte Zuführung des festen vergärbaren organischen Materials bzw. des vergärbaren organischen Materials mit hohen Feststoffanteil verhältnismäßig problematisch bzw. aufwendig. Die bislang nur in aufwendiger und somit kostspieliger Weise durchführbare dosierte und kontrollierte Zugabe von vergärbarem organischen Material mit hohem Feststoffanteil macht Biogasanlagen vor allem für kleinere und mittlere landwirtschaftliche Betriebe oftmals unrentabel.

Eine Biogasanlagen-Zuführungseinrichtung der eingangs genannten Art, von der die Erfindung ausgeht, ist aus GB 2 282 337 A bekannt. Diese bekannte Zuführungseinrichtung lässt jedoch im Hinblick auf eine funktionssichere kontrollierte und dosierte Zuführung des organischen Materials zu wünschen übrig.

Demgegenüber liegt der Erfindung das technische Problem zugrunde, eine Biogasanlagen-Zuführungseinrichtung für vergärbares organisches Material, insbesondere mit hohem Feststoffanteil anzugeben, bei der eine funktionssichere kontrollierte und dosierte Zuführung des organischen Materials möglich ist und die nichtsdestoweniger mit relativ geringem Aufwand und geringen Kosten realisierbar ist.

Zur Lösung dieses technischen Problems lehrt die Erfindung eine Biogasanlagen-Zuführungseinrichtung der eingangs genannten Art, welche dadurch gekennzeichnet ist, dass in dem Vorlagebunker zumindest eine Förder- und Mischeinrichtung für das organische Material angeordnet ist und dass die Förder- und Mischeinrichtung so ausgelegt ist, dass das organische Material zu einer Stirnseite des Vorlagebunkers förderbar ist, an welcher Stirnseite sich ein vorderer Einführabschnitt des Zuführungskanals befindet.

Es liegt im Rahmen der Erfindung, dass die erfindungsgemäße Biogasanlagen-Zuführungseinrichtung für festes vergärbares organisches Material oder für vergärbares organisches Material mit hohem Feststoffanteil von zumindest 5 Gew.-%, vorzugsweise von zumindest 15 Gew.-%, sehr bevorzugt von zumindest 20 Gew.-% eingesetzt wird. Nach sehr bevorzugter Ausführungsform der Erfindung handelt es sich bei dem vergärbaren organischen Material um Mist. Mist ist eine Mischung aus Stroh und Tierkot, wobei der Tierkot vorzugsweise von Rindern, Schweinen oder Geflügel stammt. Als vergärbares organisches Material kann in der erfindungsgemäßen Biogasanlagen-Zuführungseinrichtung auch Gras und/oder Schnittgrün und/oder Rasenschnitt verwendet werden. Fernerhin können Ernterückstände, wie Getreidereste und/oder Gemüsereste, beispielsweise Mais und/oder zerkleinerte Rüben als vergärbares organisches Material eingesetzt werden. Das vergärbare organische Material kann aber auch aus Küchenabfällen, Speiseabfällen, Fettabscheiderinhalt oder aus Schlachtnebenprodukten bestehen. - Wenn in der Biogasanlage, an der die erfindungsgemäße Biogasanlagen-Zuführungseinrichtung angeschlossen ist, auch flüssige vergärbare organische Substanzen wie Gülle (Mischung aus Tierkot und Urin) und/oder andere organisch belastete Abwässer eingesetzt werden, so werden diese flüssigen vergärbaren organischen Substanzen vorzugsweise nicht über die erfindungsgemäße Biogasanlagen-Zuführungseinrichtung der Biogasanlage zugeführt, sondern über eine gesonderte Zuführung.

Nach sehr bevorzugter Ausführungsform, der im Rahmen der Erfindung ganz besondere Bedeutung zukommt, besteht die Förder- und/oder Mischeinrichtung aus zumindest einer drehbaren Welle und sind an die Welle eine Mehrzahl von Förderund/oder Mischelementen angeschlossen. Sehr bevorzugt besteht die Förder- und/oder Mischeinrichtung aus zwei drehbaren Wellen, die zweckmäßigerweise parallel zueinander angeordnet sind. Vorzugsweise ist an jede der beiden Wellen eine Mehrzahl von Förder- und/oder Mischelementen angeschlossen. Die zumindest eine drehbare Welle ist zweckmäßigerweise parallel zu dem Zuführungskanal angeordnet. - Die Förder- und/oder Mischelemente sind vorzugsweise in radialer Richtung an eine drehbare Welle angeschlossen. Zweckmäßigerweise sind an jeder drehbaren Welle mehrere bezüglich der Längsrichtung der Welle hintereinander angeordnete Förder- und/oder Mischelemente vorgesehen. Zumindest einige der bezüglich der Längsrichtung der Welle hintereinander angeordneten Förder- und/oder Mischelemente sind dabei in verschiedene radiale Richtungen orientiert.

Erfindungsgemäß ist das organische Material mittels der Förder- und/oder Mischeinrichtung zu einer der Biogasanlage zugewandten Stirnseite des Vorlagebunkers förderbar. Für eine effektive Förderung sind zweckmäßigerweise spezielle erfindungsgemäße Förder- und/oder Mischelemente vorgesehen, die an die zumindest eine drehbare Welle angeschlossen sind. Bevorzugt handelt es sich um hakenförmige Förderund/oder Mischelemente, deren Hakenende vorzugsweise in Förderrichtung der Förder- und/oder Mischeinrichtung weist. Ein solches hakenförmiges Förder- und/oder Mischelement weist zweckmäßigerweise zunächst einen ersten Abschnitt auf, der in radialer Richtung bezüglich der Welle bzw. senkrecht oder zumindest im Wesentlichen senkrecht zur Längsrichtung der Welle an die Welle angeschlossen ist. An diesen ersten Abschnitt ist dann ein Hakenende oder Hakenabschnitt angeschlossen, welches Hakenende oder welcher Hakenabschnitt in Förderrichtung der Förder- und/oder Mischeinrichtung weist.

Nach sehr bevorzugter Ausführungsform der Erfindung ist der Vorlagebunker sich nach unten zum Zuführungskanal hin verjüngend ausgebildet. Grundsätzlich kann der Vorlagebunker dabei trichterartig ausgebildet sein. Zweckmäßigerweise weist der Vorlagebunker zumindest zwei schräg aufeinander zulaufende und unterhalb des Vorlagebunkers in die Wandungen des Zuführungskanals übergehende Bunkerwände auf. Zweckmäßigerweise schließt der Zuführungskanal unmittelbar an das untere verjüngte Ende des Vorlagebunkers an. Es liegt dabei im Rahmen der Erfindung, dass lediglich ein vorderer Einführabschnitt des Zuführungskanals in unmittelbarer Verbindung mit dem Vorlagebunker steht. Es liegt weiterhin im Rahmen der Erfindung, dass das organische Material unter Einwirkung der Schwerkraft aus dem Vorlagebunker in den Zuführungskanal bzw. in den Einführabschnitt des Zuführungskanals rutscht.

Bevorzugt weist der Zuführungskanal einen kreisrunden Querschnitt auf und vorzugsweise ist der Druckkolben mit zylindrischem Querschnitt in den Zuführungskanal eingepasst. Es liegt auch im Rahmen der Erfindung, dass der Zuführungskanal einen im Wesentlichen kreisrunden Querschnitt aufweist und der Druckkolben einen im Wesentlichen zylindrischen Querschnitt aufweist.

Zweckmäßigerweise weist der Zuführungskanal an seinem ausgabeseitigen Ende zumindest ein Absperrelement auf. Ausgabeseitiges Ende des Zuführungskanals meint vorzugsweise das Ende des Zuführungskanals, das der Biogasanlage bzw. der Zuführöffnung zur Biogasanlage zugewandt ist. Die Zuführöffnung des Zuführungskanals kann auch mit einer zusätzlichen Sperrklappe versehen sein, die sich beispielsweise unter einem bestimmten Druck des auf sie einwirkenden organischen Materials öffnet. - Bei dem erfindungsgemäßen Absperrelement handelt es sich vorzugsweise um ein Absperrventil. Der Druckkolben drückt das organische Material im geschlossenen Zustand des Absperrelementes gegen das Absperrelement. Im geöffneten Zustand des Absperrelementes wird das organische Material unter Einwirkung des Druckkolbens zur Zuführöffnung des Zuführungskanals gefördert und somit in die Biogasanlage eingespeist. Nach sehr bevorzugter Ausführungsform, der im Rahmen der Erfindung besondere Bedeutung zukommt, wird das Absperrelement mittels einer Steuer- und/oder Regeleinrichtung automatisch geöffnet und geschlossen. Die Betätigung des Absperrelementes erfolgt dabei zweckmäßigerweise nach Maßgabe des Bedarfs der Biogasanlage an dem vergärbaren organischen Material.

Vorzugsweise ist der Druckkolben hydraulisch und/oder pneumatisch betätigbar. Es liegt insbesondere im Rahmen der Erfindung, dass eine elektrohydraulische Steuerung des Druckkolbens vorgesehen ist.

Nach sehr bevorzugter Ausführungsform, der im Rahmen der Erfindung ganz besondere Bedeutung zukommt, sind der Druckkolben und/oder das Absperrelement an eine Steuer- und/oder Regeleinrichtung angeschlossen und für eine gezielte dosierte Zuführung von organischem Material betätigbar. Zweckmäßigerweise ist die Steuer- und/oder Regeleinrichtung an eine Betätigungsvorrichtung für den Druckkolben und/oder an eine Betätigungsvorrichtung für das Absperrelement angeschlossen. Es liegt im Rahmen der Erfindung, dass der Druckkolben und/oder das Absperrelement nach Maßgabe von Messwerten von der Steuer- und/oder Regeleinrichtung betätigbar sind. Dabei handelt es sich zweckmäßigerweise um in der Biogasanlage gemessene Messwerte, die der Steuer- und/oder Regeleinrichtung zugeführt und dort ausgewertet werden und nach Maßgabe dieser Messwerte erfolgt eine gezielte bzw. dosierte Zuführung des vergärbaren organischen Materials. Es liegt auch im Rahmen der Erfindung, dass mittels der Steuer- und/oder Regeleinrichtung in vorgebbaren zeitlichen Abständen vergärbares organisches Material von der erfindungsgemäßen Zuführungseinrichtung in die Biogasanlage eingeführt werden kann.

Nach einer Ausführungsform der Erfindung ist eine Wiegevorrichtung zur Ermittlung des Gewichtes des im Vorlagebunker vorhandenen organischen Materials vorgesehen. Es liegt dabei auch im Rahmen der Erfindung, dass mit der Wiegevorrichtung das Gewicht der gesamten Biogasanlagen-Zuführungseinrichtung ermittelt werden kann.

Der Erfindung liegt die Erkenntnis zugrunde, dass mit der erfindungsgemäßen Biogasanlagen-Zuführungseinrichtung eine sehr funktionssichere dosierte Zuführung von vergärbarem organischen Material, insbesondere von vergärbarem organischen Material mit hohem Feststoffanteil, zu einer Biogasanlage möglich ist. Eine vollautomatische Einbringung des vergärbaren organischen Materials ist problemlos realisierbar. Mit der erfindungsgemäßen Zuführungseinrichtung wird der Arbeitsaufwand im Vergleich zu den aus dem Stand der Technik bekannten Maßnahmen beachtlich reduziert. Im Übrigen ist mit der erfindungsgemäßen Zuführungseinrichtung die Durchmischung, die Zerkleinerung und die Zuführung des vergärbaren organischen Materials mit verhältnismäßig geringem Energieaufwand und somit mit reduzierten Energiekosten möglich. Auch insoweit zeichnet sich die Erfindung durch beachtliche Vorteile gegenüber den aus dem Stand der Technik bekannten Maßnahmen aus. Durch die geschlossene Bauweise der erfindungsgemäßen Biogasanlagen-Zuführungseinrichtung können außerdem unerwünschte Geruchsemissionen überraschend effektiv verhindert werden. Außerdem ist die Zuführungseinrichtung mit verhältnismäßig geringem Aufwand und folglich mit relativ geringen Kosten herstellbar bzw. montierbar.

Nachfolgend wird die Erfindung anhand einer lediglich ein Ausführungsbeispiel darstellenden Zeichnung näher erläutert. Es zeigen in schematischer Darstellung:
- Fig. 1: eine Seitenansicht einer erfindungsgemäßen Zuführungseinrichtung im Schnitt und
- Fig. 2: einen Schnitt A-A durch den Gegenstand nach Fig. 1.

Die Figuren zeigen eine erfindungsgemäße Biogasanlagen-Zuführungseinrichtung 1 für die Einführung von vergärbarem organischen Material 2 in eine Biogasanlage bzw. in den Gärraum 3 einer Biogasanlage. Zunächst ist ein Vorlagebunker 4 zur Aufnahme des organischen Materials 2 vorgesehen. In dem Vorlagebunker 4 ist eine Förder- und Mischeinrichtung 5 für das organische Material 2 angeordnet, die vorzugsweise und im Ausführungsbeispiel aus zwei drehbaren Wellen 6, 7 besteht, an welche drehbaren Wellen 6, 7 eine Mehrzahl von Förder- und Mischelementen 8 angeschlossen sind. Zweckmäßigerweise und im Ausführungsbeispiel sind die beiden drehbaren Wellen 6, 7 parallel zueinander angeordnet. Die Förder- und Mischelemente 8 stehen zweckmäßigerweise und im Ausführungsbeispiel in radialer Richtung von einer drehbaren Welle 6, 7 ab. Bezüglich der Längsrichtung der Welle 6, 7 sind mehrere Förder- und Mischelemente 8 hintereinander angeordnet, die jedoch vorzugsweise in verschiedene radiale Richtungen weise. Die Förder- und Mischeinrichtung 5 ist so ausgelegt und die Förder- und Mischelemente 8 sind so ausgebildet, dass das organische Material 2 zu einer Stirnseite des Vorlagebunkers 4 förderbar ist, an welcher Stirnseite sich zweckmäßigerweise ein vorderer Einführabschnitt 9 eines Zuführungskanals 10 befindet. Nach sehr bevorzugter Ausführungsform der Erfindung und im Ausführungsbeispiel sind die Förder- und Mischelemente 8 hakenförmig ausgebildet. Die Förder- und Mischelemente 8 weisen dabei zweckmäßigerweise einen ersten Abschnitt 11 auf, der in radialer Richtung an eine drehbare Welle 6, 7 angeschlossen ist bzw. senkrecht zur Längsrichtung der drehbaren Welle 6, 7 angeordnet ist. An diesen ersten Abschnitt 11 eines Förder- und Mischelementes 8 schließt vorzugsweise ein Hakenende 12 an, das bevorzugt in Förderrichtung bzw. in Richtung der Stirnseite des Vorlagebunkers 4 ausgerichtet ist, an der sich der vordere Einführabschnitt 9 befindet. Mit der erfindungsgemäßen Förder- und Mischeinrichtung ist im Übrigen eine überraschend effektive und funktionssichere Zerkleinerung des vergärbaren organischen Materials 2 möglich. Diese Zerkleinerung ist besonders wirksam, wenn es sich bei dem vergärbaren organischen Material 2 um Mist handelt.

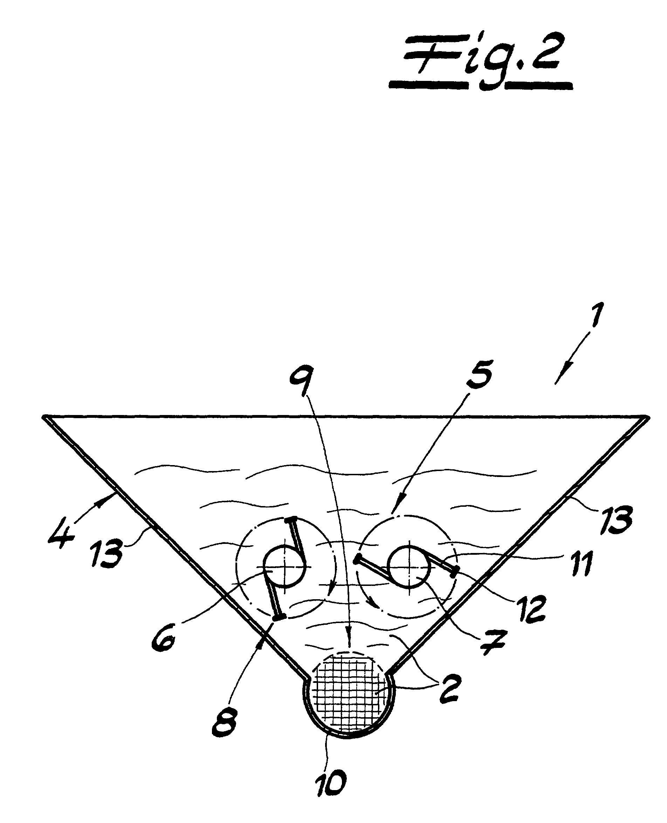

Nach sehr bevorzugter Ausführungsform und im Ausführungsbeispiel (Fig. 2) ist der Vorlagebunker 4 sich nach unten zum Zuführungskanal 10 hin verjüngend ausgebildet. Im Ausführungsbeispiel sind zwei schräg aufeinander zulaufende und unten in den Zuführungskanal 10 übergehende Bunkerwände 13 vorgesehen. Der Zuführungskanal 10 schließt also unmittelbar an das verjüngte Ende des Vorlagebunkers 4 an. Zweckmäßigerweise ist zumindest der vordere Einführabschnitt 9 des Zuführungskanals 10 direkt mit dem Vorlagebunker 4 verbunden. Das organische Material 2 fällt aus dem Vorlagebunker 4 vorzugsweise unter Einwirkung der Schwerkraft im Bereich des vorderen Einführabschnittes 9 in den Zuführungskanal 10.

In dem Zuführungskanal 10 ist ein Druckkolben 14 angeordnet, unter dessen Einwirkung das in dem Zuführungskanal 10 aufgenommene organische Material 2 der Biogasanlage zuführbar ist. Vorzugsweise und im Ausführungsbeispiel weist der Zuführungskanal 10 einen kreisrunden Querschnitt auf (Fig. 2) und ist der Druckkolben 14 mit zylindrischem Querschnitt in diesen Zuführungskanal 10 eingepasst.

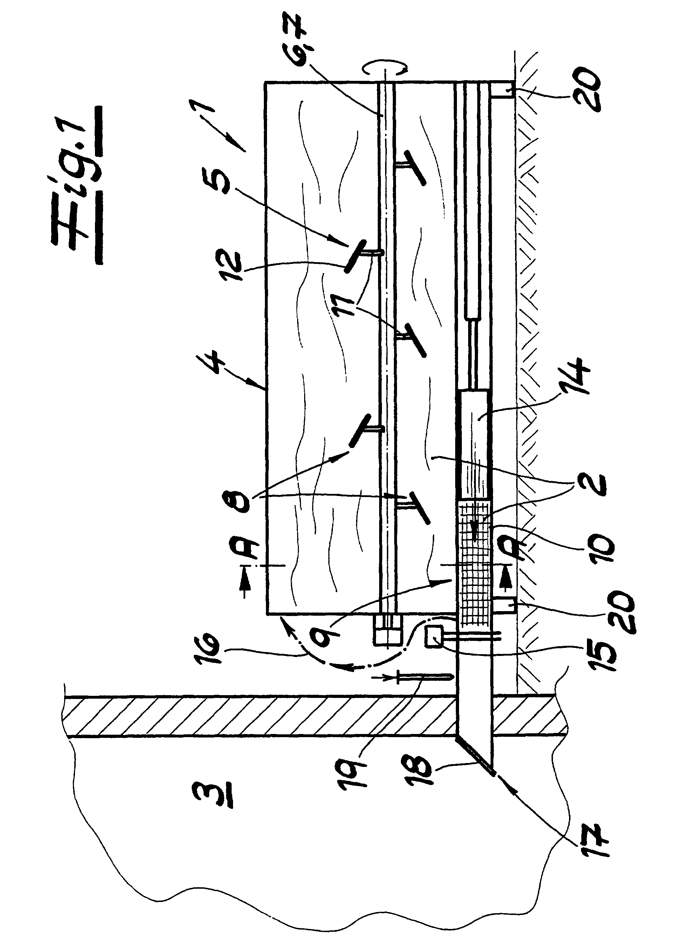

Es liegt im Rahmen der Erfindung, dass der Zuführungskanal 10 an seinem ausgabeseitigen Ende ein Absperrelement aufweist, das vorzugsweise und im Ausführungsbeispiel (Fig. 1) als Absperrventil 15 ausgebildet ist. Im geschlossenen Zustand des Absperrventils 15 drückt der Druckkolben 14, der vorzugsweise hydraulisch betätigt wird, das organische Material 2 im Zwischenraum zwischen Druckkolben 14 und Absperrventil 15 zusammen. Zweckmäßigerweise und im Ausführungsbeispiel (Fig. 1) ist vor dem Absperrventil 15 eine Abführleitung 16 an den Zuführungskanal 10 angeschlossen, über welche Abführleitung 16 bei der Kompression des organischen Materials 2 eventuell freiwerdende Flüssigkeit abgeführt werden kann. Wird das Absperrventil 15 geöffnet, so drückt der Druckkolben 14 das organische Material 2 in den Gärraum 3 der Biogasanlage. Zweckmäßigerweise und im Ausführungsbeispiel ist an der dem Gärraum 3 zugeordneten Zuführöffnung 17 des Zuführungskanals 10 eine Sperrklappe 18 vorgesehen, die unter einem bestimmten Druck des einwirkenden organischen Materials 2 geöffnet wird, so dass das organische Material 2 in den Gärraum 3 der Biogasanlage gelangt. Anschließend wird das Absperrventil 15 wieder geschlossen. Zweckmäßigerweise und im Ausführungsbeispiel ist außerdem ein Handschieber 19 vorgesehen, mit dem der Zuführungskanal 10 verschlossen werden kann, wenn das Absperrventil 15 versagen sollte.

Nach sehr bevorzugter Ausführungsform der Erfindung ist der Druckkolben 14 und ist das Absperrventil 15 an eine nicht dargestellte Steuer- und/oder Regeleinrichtung angeschlossen. Der Druckkolben 14 sowie das Absperrventil 15 sind von dieser Steuer- und/oder Regeleinrichtung für eine gezielte dosierte Zuführung des organischen Materials 2 betätigbar. Vorzugsweise erfolgt diese Betätigung des Druckkolbens 14 und/oder des Absperrventils 15 dabei nach Maßgabe von Messwerten, die der Steuer- und/oder Regeleinrichtung zugeführt werden und dort ausgewertet werden.

In Fig. 1 wurden im Übrigen Elemente 20 einer Wiegevorrichtung zur Ermittlung des Gewichtes des im Vorlagebunker 4 vorhandenen organischen Materials 2 angedeutet. Mit dieser Wiegeeinrichtung kann zweckmäßigerweise und im Ausführungsbeispiel das Gewicht der gesamten erfindungsgemäßen Biogasanlagen-Zuführungseinrichtung 1 ermittelt werden.

## Patentansprüche

1. Biogasanlagen-Zuführungseinrichtung (1) für vergärbares organisches Material (2),
wobei ein Vorlagebunker (4) zur Aufnahme des organischen Materials (2) vorgesehen ist,
wobei unterhalb des Vorlagebunkers (4) zumindest ein Zuführungskanal (10) für eine Aufnahme des organischen Materials (2) aus dem Vorlagebunker (4) vorgesehen ist,
wobei in dem Zuführungskanal (10) ein Druckkolben (14) angeordnet ist, unter dessen Einwirkung das in dem Zuführungskanal (10) aufgenommene organische Material (2) der Biogasanlage zuführbar ist, **dadurch gekennzeichnet,**
**dass** in dem Vorlagebunker (4) zumindest eine Förder- und Mischeinrichtung (5) für das organische Material (2) angeordnet ist und
**dass** die Förder- und Mischeinrichtung (5) so ausgelegt ist, dass das organische Material (2) zu einer Stirnseite des Vorlagebunkers (4) förderbar ist, an welcher Stirnseite sich ein vorderer Einführabschnitt (9) des Zuführungskanals (10) befindet.

2. Biogasanlagen-Zuführungseinrichtung nach Anspruch 1, **dadurch gekennzeichnet, dass** die Förder- und Mischeinrichtung (5) aus zumindest einer drehbaren Welle (6, 7) besteht und wobei an die Welle (6, 7) eine Mehrzahl von Förder- und/oder Mischelementen (8) angeschlossen ist.

3. Biogasanlagen-Zuführungseinrichtung nach einem der Ansprüche 1 oder 2, **dadurch gekennzeichnet, dass** das organische Material (2) mittels der Förder- und Mischeinrichtung (5) zu einer der Biogasanlage zugewandten Stirnseite des Vorlagebunkers (4) förderbar ist.

4. Biogasanlagen-Zuführungseinrichtung nach einem der Ansprüche 1 bis 3, **dadurch gekennzeichnet, dass** der Vorlagebunker (4) sich nach unten zum Zuführungskanal (10) hin verjüngend ausgebildet ist.

5. Biogasanlagen-Zuführungseinrichtung nach einem der Ansprüche 1 bis 4, **dadurch gekennzeichnet, dass** der Zuführungskanal (10) einen kreisrunden Querschnitt aufweist und der Druckkolben (14) mit zylindrischem Querschnitt in den Zuführungskanal (10) eingepasst ist.

6. Biogasanlagen-Zuführungseinrichtung nach einem der Ansprüche 1 bis 5, **dadurch gekennzeichnet, dass** der Zuführungskanal (10) an seinem ausgabeseitigen Ende zumindest ein Absperrelement aufweist.

7. Biogasanlagen-Zuführungseinrichtung nach einem der Ansprüche 1 bis 6, **dadurch gekennzeichnet, dass** der Druckkolben (14) hydraulisch und/oder pneumatisch betätigbar ist.

8. Biogasanlagen-Zuführungseinrichtung nach einem der Ansprüche 1 bis 7, **dadurch gekennzeichnet, dass** der Druckkolben (14) und/oder das Absperrelement an eine Steuer- und/oder Regeleinrichtung angeschlossen sind und für eine gezielte dosierte Zuführung von organischem Material (2) betätigbar ist.

9. Biogasanlagen-Zuführungseinrichtung nach einem der Ansprüche 1 bis 8, **dadurch gekennzeichnet, dass** der Druckkolben 14 und/oder das Absperrelement nach Maßgabe von Messwerten von der Steuer- und/oder Regeleinrichtung betätigbar ist.

10. Biogasanlagen-Zuführungseinrichtung nach einem der Ansprüche 1 bis 9, **dadurch gekennzeichnet, dass** eine Wiegevorrichtung zur Ermittlung des Gewichtes des im Vorlagebunker (4) vorhandenen organischen Materials (2) vorgesehen ist.

## Claims

1. A biogas installation feeder device (1) for fermentable organic material (2),
wherein a collecting hopper (4) is provided for receiving the organic material (2),
wherein at least one feed duct (10) for receiving the organic material (2) from the collecting hopper (4) is provided under the collecting hopper (4),
wherein a pressure piston (14), under the action of which the organic material (2) which is received in the feed duct (10) can be fed to the biogas installation, is disposed in the feed duct (10), **characterised in that**
at least one conveying and mixing device (5) for the organic material (2) is disposed in the collecting hopper (4), and
that the conveying and mixing device (5) is designed so that the organic material (2) can be conveyed to an end face of the collecting hopper (4) at which end face a front input section (9) of the feed duct (10) is situated.

2. A biogas installation feeder device according to claim 1, **characterised in that** the conveying and mixing device (5) consists of at least one shaft (6, 7) which can be rotated, wherein a multiplicity of feeding and/or mixing elements (8) is attached to the shaft (6, 7).

3. A biogas installation feeder device according to either one of claims 1 or 2, **characterised in that** the organic material (2) can be conveyed by means of the conveying and mixing device (5) to an end face of the collecting hopper (4) facing the biogas installation.

4. A biogas installation feeder device according to any one of claims 1 to 3, **characterised in that** the collecting hopper (4) is constructed so that it tapers downwards towards the feed duct (10).

5. A biogas installation feeder device according to any one of claims 1 to 4, **characterised in that** the feed duct (10) has a circular cross-section and the cross-section of the cylindrical pressure piston (14) is fitted into the feed duct (10).

6. A biogas installation feeder device according to any one of claims 1 to 5, **characterised in that** the feed duct (10) has at least one shut-off element at its output end.

7. A biogas installation feeder device according to any one of claims 1 to 6, **characterised in that** the pressure piston (14) can be operated hydraulically and/or pneumatically.

8. A biogas installation feeder device according to any one of claims 1 to 7, **characterised in that** the pressure piston (14) and/or the shut-off element are connected to a control and/or regulating device and can be operated for a specific, metered feed of organic material (2).

9. A biogas installation feeder device according to any one of claims 1 to 8, **characterised in that** the pressure piston (14) and/or the shut-off element can be operated by the control and/or regulating device according to measured values.

10. A biogas installation feeder device according to any one of claims 1 to 9, **characterised in that** a weighing device is provided for determining the weight of the organic material (2) which is present in the collecting hopper (4).

## Revendications

1. Dispositif d'alimentation d'une unité de production de biogaz (1) pour matière organique fermentescible (2), un collecteur (4) étant prévu pour recevoir la matière organique (2), au moins un canal d'alimentation (10) pour recevoir la matière organique (2) du collecteur (4) étant prévu au-dessous de ce dernier, un piston de compression (14), sous l'effet duquel la matière organique (2) reçue dans le canal d'alimentation (10) peut être envoyée à l'unité de production de biogaz, étant disposé dans le canal d'alimentation (10), **caractérisé en ce qu'**au moins un dispositif de transport et de mélange (5) pour la matière organique (2) est disposé dans le collecteur (4), et **en ce que** le dispositif de transport et de mélange (5) est conçu de sorte que la matière organique (2) peut être refoulée en direction d'un côté frontal du collecteur (4), côté frontal sur lequel se situe une section d'admission avant (9) du canal d'alimentation (10).

2. Dispositif d'alimentation d'une unité de production de biogaz suivant la revendication 1, **caractérisé en ce que** le dispositif de transport et de mélange (5) se compose d'au moins un arbre rotatif (6, 7), une multiplicité d'éléments de transport et de mélange (8) étant raccordée à l'arbre (6, 7).

3. Dispositif d'alimentation d'une unité de production de biogaz suivant l'une des revendications 1 et 2, **caractérisé en ce que** la matière organique (2) peut être refoulée en direction d'un côté frontal, tourné vers l'unité de production de biogaz, du collecteur (4) au moyen du dispositif de transport et de mélange (5).

4. Dispositif d'alimentation d'une unité de production de biogaz suivant l'une des revendications 1 à 3, **caractérisé en ce que** le collecteur (4) a une réalisation rétrécie vers le bas en direction du canal d'alimentation (10).

5. Dispositif d'alimentation d'une unité de production de biogaz suivant l'une des revendications 1 à 4, **caractérisé en ce que** le canal d'alimentation (10) présente une section transversale circulaire et le piston de compression (14) est encastré par sa section transversale cylindrique dans le canal d'alimentation (10).

6. Dispositif d'alimentation d'une unité de production de biogaz suivant l'une des revendications 1 à 5, **caractérisé en ce que** le canal d'alimentation (10) présente au moins un élément d'arrêt sur son extrémité côté décharge.

7. Dispositif d'alimentation d'une unité de production de biogaz suivant l'une des revendications 1 à 6, **caractérisé par** une possibilité d'actionnement hydraulique et/ou pneumatique du piston de compression (14).

8. Dispositif d'alimentation d'une unité de production de biogaz suivant l'une des revendications 1 à 7, **caractérisé en ce que** le piston de compression (14) et/ou l'élément d'arrêt sont raccordés à un dispositif de commande et/ou de régulation et sont actionnables pour une alimentation dosée rationnelle en matière organique (2).

9. Dispositif d'alimentation d'une unité de production de biogaz suivant l'une des revendications 1 à 8, **caractérisé en ce que** le piston de compression 14 et/ou l'élément d'arrêt sont actionnables par le dispositif de commande et/ou de régulation conformément à des valeurs de mesure.

10. Dispositif d'alimentation d'une unité de production de biogaz suivant l'une des revendications 1 à 9, **caractérisé en ce qu'**un dispositif de pesée est prévu pour déterminer le poids de la matière organique (2) présente dans le collecteur (4).
